(19) Europäisches Patentamt
European Patent Office
Office européen des brevets

(11) Publication number: **0 102 687**
**B1**

(12) # EUROPEAN PATENT SPECIFICATION

(45) Date of publication of patent specification: **15.03.89**

(51) Int. Cl.$^4$: **C 07 C 131/00,**
C 07 D 277/40, C 07 D 501/20

(21) Application number: **83303183.4**

(22) Date of filing: **02.06.83**

(54) **Process for the preparation of a 4-halo-2-hydroxyimino-acetoacetic acid ester.**

(30) Priority: **03.06.82 GB 8216251**

(43) Date of publication of application:
**14.03.84 Bulletin 84/11**

(45) Publication of the grant of the patent:
**15.03.89 Bulletin 89/11**

(84) Designated Contracting States:
**AT BE CH DE FR GB IT LI NL SE**

(56) References cited:
**ORGANIC REACTIONS, vol. VII, Chapter 6, J. Wiley, 1967, NEW YORK (US), O. TOUSTER: "The nitrosation of aliphatic carbon atoms", pp. 327-355**

(73) Proprietor: **GLAXO GROUP LIMITED**
**Clarges House 6-12 Clarges Street**
**London W1Y 8DH (GB)**

(72) Inventor: **Turner, Michael Robert**
**2 Lewins Way**
**Cippenham near Slough Buckinghamshire (GB)**
Inventor: **Miller, Thomas**
**37 Moorhall Road**
**Harefield Middlesex (GB)**

(74) Representative: **Holmes, Michael John et al**
**Frank B. Dehn & Co. European Patent Attorneys**
**Imperial House 15-19 Kingsway**
**London, WC2B 6UZ, (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 687 B1

**Description**

This invention relates to improvements in or relating to the manufacture of antibiotics. More particularly, it relates to a novel process for the preparation of intermediates of value in the synthesis of certain β-lactam antibiotics, for example, cephalosporins.

A number of antibiotic cephalosporin compounds are now known which have a 2-(2-aminothiazol-4-yl)-2-(substituted oxyimino)acetamido side chain in the 7-position. It is particularly desirable that the 2-(substituted oxyimino) group should be in the *syn* isomeric form, the *syn*-isomers exhibiting greater antibacterial activity than their corresponding anti-isomers. The *syn*-configuration of the 7-side chain is defined by the configuration of the oxime substituent with respect to the carboxamido group and may be represented thus:—

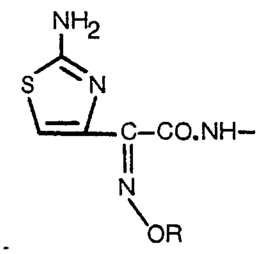

where R is the oxyimino substituent. One such antibiotic, (6R,7R) - 7 - [(Z) - 2 - (2 - aminothiazol - 4 - yl) - 2 - (2 - carboxyprop - 2 - oxyimino)acetamido] - 3 - (1 - pyridiniummethyl)ceph - 3 - em - 4 - carboxylate known by the approved name "ceftazidime", is described *inter alia* in our US Patent Specification No. 4 258 041 and details of its preparation are given therein.

One general method for preparing cephalosporin antibiotics having a *syn* 2-(2-aminothiazol-4-yl)-2-(substituted oxyimino)acetamido side-chain at the 7-position involves building up the 7-side-chain through a series of reactions to form the desired (Z)-2-(2-aminothiazol-4-yl)-2-(substituted oxyimino)acetic acid (the side-chain acid) or a reactive derivative thereof followed by coupling with the 7β-amino cephalosporin nucleus. The side-chain acid is in the form of a *syn*-isomer and this is indicated by the Z notation. A series of such reactions for the preparation of protected forms of the side-chain acids for ceftazidime and related compounds are described in Preparations 1 to 9 of the above-mentioned US Patent Specification.

In the first stage of these sequences of reactions, ethyl acetoacetate is reacted with sodium nitrite in acetic acid to give ethyl (Z)-2-hydroxyiminoacetoacetate which is subsequently reacted with sulphuryl chloride to form ethyl (Z)-4-chloro-2-hydroxyiminoacetoacetate and then thiourea to form ethyl (Z)-2-(2-aminothiazol-4-yl)-2-hydroxyiminoacetate. This compound is then treated to yield the desired side-chain acid, e.g. by introducing the substituent on the oxyimino group. However, the yields of the ethyl (Z)-4-chloro-2-hydroxyiminoacetoacetate intermediate and of ethyl (Z)-2-(2-aminothiazol-4-yl)-2-hydroxyimino-acetate obtained in this reaction sequence are generally unsatisfactory for commercial production and it is usually necessary to isolate the 4-chloro intermediate in the process.

European Patent Application Specification No. 45005 describes a process for the preparation of esters of (Z)-2-(2-aminothiazol-4-yl)-2-methoxyiminoacetic acid by oximating (i.e. nitrosating) a 4-chloro-acetoacetic acid ester, reacting the resulting 4-chloro-2-hydroxyiminoacetoacetic acid ester, without isolation, with thiourea to give a (Z)-2-(2-aminothiazol-4-yl)-2-hydroxyiminoacetic acid ester and then methylating this compound to give the desired product. The nitrosation reaction is effected by reacting the starting compound with an alkali metal nitrite (the use of sodium nitrite is exemplified) in the presence of acetic acid. However, we have found that the yield of (Z)-2-(2-aminothiazol-4-yl)-2-hydroxyiminoacetic acid ester obtained in such a process is not particularly satisfactory.

British Patent Specification No. 1576625 generically described *inter alia* the nitrosation of a 4-halo-acetoacetic acid in which the carboxyl group is protected, to form a mixture of *syn* and anti isomers of the corresponding protected 4-halo-2-hydroxyiminoacetoacetic acid. According to this specification, the nitrosation reaction may be effected using nitrous acid, an alkali metal nitrite or a lower alkyl nitrite, although only the use of sodium nitrite is exemplified. It is indicated that, when an alkyl nitrite is to be used, the reaction should be carried out in the presence of a strong base.

Japanese Published Patent Application No. 56-100772 describes a process for the preparation of 2-(2-aminothiazol-4-yl)-2-alkoxyiminoacetic acid esters by reacting an acetoacetic acid ester with an alkyl nitrite in the presence of a mineral acid to give a 2-hydroxyiminoacetoacetic acid ester which is then isolated. The isolated compound is then reacted with a dialkyl sulphate to give the corresponding 2-alkoxyiminoaceto-acetate ester, which is itself isolated and reacted with a halogen to form a 4-halo-2-alkoxyiminoacetoacetic acid ester. Finally, the isolated halogenated compound is reacted with thiourea to give the desired final product. There is no indication in this publication as to whether the final product is in the form of a *syn* or *anti*-isomer, or a mixture of such isomers. Moreover, it is to be noted that it is necessary to isolate the intermediates produced in each stage of the reaction sequence described in Japanese Published Patent Application No. 56-100772 and, indeed, a different solvent system is used for each stage. Such a process would be inconvenient and uneconomical to operate on a manufacturing scale.

We have now discovered that 4-halo-2-hydroxyiminoacetoacetic acid esters, predominantly in the *syn*-isomeric form, may be obtained in unexpectedly high yield by reaction of 4-halo-acetoacetic acid esters (which are readily available commercially) with an alkyl nitrite under acidic conditions. The 4-halo-hydroxyiminoacetoacetic acid ester can then be reacted with thiourea to form the desired 2-(2-aminothiazol-4-yl)-2-hydroxyiminoacetic acid ester in substantially pure *syn*-isomeric form, which is obtained in correspondingly high yield. Furthermore, we have found that high yields of the *syn* 2-(2-aminothiazol-4-yl)-2-hydroxyiminoacetic acid ester may surprisingly be obtained even when the reaction sequence is effected without isolation of the intermediate 4-halo-2-hydroxyiminoacetic acid ester. We have found that even on large-scale operation the new process enables the preparation of *syn* 2-(2-aminothiazol-4-yl)-2-hydroxyiminoacetic acid esters in high yield and in a simple and economic manner. Thus, the new process has considerable advantages in manufacturing terms.

Thus, according to one aspect of the invention there is provided a process for the preparation of a 4-halo-2-hydroxyiminoacetoacetic acid ester in which a 4-halo-acetoacetic acid ester is reacted with an alkyl nitrite under acidic conditions.

The starting 4-halo-acetoacetic acid ester for the process of the invention is generally an alkyl ester such as a $C_{1-4}$ alkyl ester e.g. an ethyl ester. The 4-halo substituent may conveniently be chlorine or bromine. The alkyl nitrite starting material is generally a $C_{1-6}$ alkyl nitrite such as, for example, methyl nitrite, butyl nitrite, iso-amyl nitrite and preferably isopropyl nitrite.

Where the 4-halo-2-hydroxyiminoacetoacetic acid ester prepared according to the invention is subsequently converted into a side chain acid ester containing a further blocked carboxyl group —COOR′ (for example in the substituted oxyimino moiety, as in protected forms of the ceftazidime side chain acid) it may be necessary, prior to a coupling reaction with an amino β-lactam, to remove the original ester group by selective hydrolysis whilst retaining the carboxyl blocking group R′. It will be appreciated that in such instances the esterifying group in the starting 4-haloacetoacetic acid ester is desirably one which may readily be removed by a method which does not affect the carboxyl blocking group R′.

The nitrosation reaction of the invention is effected under acidic conditions e.g. in the presence of a mineral acid such as hydrochloric or sulphuric acid. The reaction may conveniently be effected in the presence of one or more organic solvents and/or an excess of the alkyl nitrite. Examples of solvents which may be employed include hydrocarbons such as a light petroleum, toluene or benzene, ethers such as di-isopropyl ether, diethyl ether or dioxan, esters such as ethyl acetate, and/or alcohols such as isopropyl alcohol. Isopropyl alcohol is a particularly convenient solvent when isopropyl nitrite is used, as isopropyl alcohol is a product of the reaction. The solvent system is generally chosen so that optimal solubility for the starting materials is obtained and a mixture of solvents may be employed. The solvents used should be liquid at the temperature selected for the reaction which may, for example, be from −50 to +150°C, preferably from 0 to +50°C.

The 4-halo-2-hydroxyiminoacetoacetic acid ester, may if desired be isolated from the reaction medium and optionally crystallized prior to a subsequent reaction with thiourea to form the desired 2-(2-aminothiazol-4-yl)-2-hydroxyiminoacetic acid ester substantially in the *syn*-isomeric form. However, isolation is not essential and it may be more convenient to react the 4-halo compound directly, without isolation, with thiourea to give the desired aminothiazolyl compound. Thus, it will be noted that the desired side-chain acid ester is obtained substantially in the *syn* isomeric form whether or not the 4-halo intermediate is isolated.

The reaction of the 4-halo-2-hydroxyiminoacetoacetic acid ester prepared according to the invention with thiourea is preferably carried out in the presence of a base, such as pyridine or N,N-dimethylaniline. If the reaction is effected without isolation of the 4-halo intermediate, it is generally necessary to remove any nitrosating species from the reaction medium which may remain after the nitrosation step in order to prevent these species from reacting with the *syn* 2-(2-aminothiazol-4-yl)-2-hydroxyiminoacetic acid ester. As such nitrosating species are generally volatile compounds they may conveniently be removed by passing a stream of an inert gas, such as nitrogen, through the reaction medium. It will be appreciated that where the 4-halo product is isolated prior to reaction with thiourea, then any nitrosating species will be removed in the isolation step.

The reaction with thiourea is conveniently effected in the presence of one or more organic solvents such as esters, ethers and/or alcohols, for example as described above for the reaction with the alkyl nitrite, optionally in admixture with water. It is particularly convenient to use one or more alcohols as the solvent since this facilitates the subsequent crystallisation of the desired final product. It is convenient to effect the reaction with thiourea at a temperature of −50 to +150°C, preferably from 0 to +50°C.

The *syn* 2-(2-aminothiazol-4-yl)-2-hydroxyiminoacetic acid ester produced by the process may be recovered from the reaction medium by any convenient means, such as by crystallisation.

As indicated above, cephalosporin antibiotic compounds having a *syn* substituted oxyimino group in the 7-side-chain have been found to exhibit enhanced antibacterial properties. It is generally desirable to employ the *syn*-isomeric form of the side-chain acid in their preparation. The process of the invention has been found to enhance greatly the yields of the *syn* isomers of both the 4-halo intermediate and the side chain acid ester. In particular, the 4-halo intermediate is obtained predominantly as its *syn* isomer e.g. containing at least 75% and preferably 85%, of the *syn*-isomer. When the 4-halo intermediate is isolated in crystalline form, it has been found to be substantially in the *syn*-isomeric form. In order to maximise the

recovery of the 2-(2-aminothiazol-4-yl)-2-hydroxyiminoacetic acid ester, this compound is preferably isolated as a free base as this assists in the crystallisation of the *syn* isomer, leaving any *anti* isomer in solution in the solvents normally used.

The process of the invention and the subsequent reaction with thiourea enable *syn* 2-(2-aminothiazol-4-yl)-2-hydroxyiminoacetic acid esters to be obtained in high yield. The product may then be subjected to a further series of reactions to yield a compound which may, for example, be coupled to a 7β-amino cephalosporin to give an antibiotic cephalosporin such as ceftazidime, for example as described in our US Patent Specification No. 4258041. *Syn* 2-(2-aminothiazol-4-yl)-2-hydroxyiminoacetic acid esters prepared according to the invention may also be used in the preparation of other β-lactam antibiotics, e.g. cephalosporin antibiotics such as cefotaxime, cefmenoxime, ceftriaxon, eftizoxime and cefodizime, as well as monocyclic β-lactam antibiotics such as azthreonam.

The process according to the invention will be illustrated by the following non-limiting examples. In the examples, all temperatures are in °C. HPLC is high performance liquid chromatography.

## Example 1

a) Ethyl (Z)-4-chloro-2-hydroxyiminoacetoacetate

An 8M solution of hydrogen chloride in ethanol (0.4 ml) was added to a stirred solution of butyl nitrite (11.45 g) and ethyl 4-chloroacetoacetic (16.49 g) in diethyl ether (40 ml) and the mixture was stirred overnight. After 17 h, thin layer chromatography (petrol:ethyl acetate 1:1) indicated complete conversion of ethyl 4-chloroacetoacetate (*R*f 0.72) into the *title compound (R*f 0.56) and its *anti* isomer (running slightly slower). The solution was washed with water, saturated aqueous sodium chloride adjusted to pH6 with sodium bicarbonate, and saturated sodium chloride; dried over magnesium sulphate, and concentrated at 70°/0.5 mmHg to give the title compound (19.7 g) as an oil. N.m.r. (DMSO-$d_6$) showed Cl$CH_2$CO signals at τ5.01 (*syn* isomer, 1.68H) and τ5.32 (*anti* isomer, 0.32H). After crystallisation of the oil the *syn:anti* ratio was shown by n.m.r. to be about 60:1.

b) Ethyl (Z)-2-(2-aminothiazol-4-yl)-2-hydroxyiminoacetate

Ethyl (Z)-4-chloro-2-hydroxyiminoacetoacetate (18.6 g) was stirred with isopropyl alcohol (45 ml) to give a clear solution. N,N-Dimethylaniline (13.5 ml) and thiourea (7.6 g) were added, and the mixture was stirred for 17 h, its temperature reaching a maximum of 40° after ½ h. The product was collected by filtration, washed with two portions of isopropyl alcohol (total 15 ml), and dried for 3 h *in vacuo* at 40° to afford the title compound as the substantially pure *syn* isomer (14.9 g) as a powder, m.p. 190—196°; λmax (ethanol) 228 nm ($E^{1\%}_{1cm}$ 735); n.m.r. (DMSO-$d_6$) τ values include −1.62 (s, OH), 2.83 (s, $NH_2$), 3.13 (s, ring proton), 5.66 and 8.71 (q and t, ethyl ester protons). A second crop was obtained by treating the mother liquor with water (160 ml); it was washed with water and dried overnight *in vacuo* at 40° to give the title compound (2.4 g), m.p. 187—194°; λmax (ethanol) 228 nm ($E^{1\%}_{1cm}$ 706).

## Example 2

Ethyl (Z)-2-(2-aminothiazol-4-yl)-2-hydroxyiminoacetate

Conc. hydrochloric acid (0.9 ml) was added to a mixture of ethyl 4-chloroacetoacetate (82.3 g), isopropyl nitrite (71 ml) and isopropyl alcohol (100 ml) with stirring and ice cooling. After an initial rise to 30° the solution was allowed to stand overnight at room temperature. After removal of excess nitrosating agents in a stream of nitrogen for 90 min., N,N-dimethylaniline (64 ml) and thiourea (38 g) were added, and the mixture was stirred with ice cooling for 2½ h at 20—25°. The product was collected by filtration after cooling to −10°, washed with water, then with isopropyl alochol, and dried overnight *in vacuo* at 40° to give the title compound as the substantially pure *syn* isomer (76.6 g); m.p. 192—197°; λmax (ethanol) 228 nm ($E^{1\%}_{1cm}$ 728); n.m.r. (DMSO-$d_6$) resembles that of Example 1.

## Example 3

Ethyl (Z)-2-(2-aminothiazol-4-yl)-2-hydroxyiminoacetate

Ethyl 4-chloroacetoacetate (82.3 g), isopropyl alcohol (100 ml), isopropyl nitrite (55 ml) and conc. HCl (3.0 ml) were mixed at 10° with ice cooling. The mixture was allowed to warm to room temperature. After 21 h excess nitrosating agents were removed in a stream of nitrogen, for 90 mins.

To this solution was added a solution of pyridine (43 ml) and thiourea (38 g) in distilled water (200 ml) over 2.25 h at 22—24°, with stirring and cold-water cooling. The resulting mixture was stirred for 3 h at 21—22° and left to stand overnight at 20°. The product was collected by filtration, washed with isopropyl alcohol/water (60 ml, 2:3), water (80 ml), and twice with isopropyl alcohol (total 85 ml), then dried overnight *in vacuo* at 40° to yield the title compound as the substantially pure *syn* isomer (85.1 g) as a solid; m.p. 194—198°; λmax (ethanol) 228 nm ($E^{1\%}_{1cm}$ 728); n.m.r. (DMSO-$d_6$) resembles that of Example 1.

## Example 4

Ethyl (Z)-2-(2-aminothiazol-4-yl)-2-hydroxyiminoacetate

A mixture of ethyl 4-chloroacetoacetate (82.3 g), isopropyl alcohol (100 ml), isopropyl nitrite (55 ml) and a solution of conc. hydrochloric acid (3.0 ml) in $H_2O$ (16 ml) was kept at 35° for 3 h after which nitrogen was bubbled through the mixture for 90 min to remove excess nitrosating agents. A solution of pyridine

(43 ml) and thiourea (38.0 g) in distilled water (180 ml) was added over 2.75 h at 22—24°, with stirring and cold-water cooling; the transfer was completed with water (24 ml). The mixture was stirred overnight at 22° and the product was collected by filtration, washed with isopropyl alcohol/$H_2O$ (40 ml, 2:3), water (80 ml) and isopropyl alcohol (70 ml), dried overnight *in vacuo* at 40° to give the title compound, as the substantially pure *syn* isomer (83.7 g) as a solid; m.p. 191—194°; λmax (ethanol) 228 nm ($E_{1\,cm}^{1\%}$ 744); n.m.r. resembles that of Example 1.

Example 5

Ethyl (Z)-2-(2-aminothiazol-4-yl)-2-hydroxyiminoacetate

A mixture of iso-amyl nitrite (21 ml 18.3 g), isopropyl alcohol (30 ml), ethyl-4-chloroacetoacetate (24.7 g) and conc. hydrochloric acid (0.8 ml) was cooled in ice-water and allowed to stand overnight at 10—21°. After removal of excess nitrosating agents with nitrogen a solution of pyridine (13 ml) and thiourea (11.5 g) in water (66 ml) was added over 25 min at 20±5°. The mixture was stirred for 4 h then allowed to stand overnight. The product was collected by filtration, washed with isopropyl alcohol/water (60 ml, 2:3) and isopropyl alcohol (30 ml), and dried overnight *in vacuo* at 40° to yield the title compound as the substantially pure *syn* isomer (23.3 g); λmax 228 nm ($E_{1\,cm}^{1\%}$ 693); n.m.r. (DMSO-$d_6$) resembles that of Example 1.

Example 6

Ethyl (Z)-2-(2-aminothiazol-4-yl)-2-hydroxyiminoacetate

Ethyl 4-chloroacetoacetate (82.3 g), di-isopropyl ether (57 ml), isopropyl nitrite (57 ml) and conc. hydrochloric acid (0.88 ml) were mixed at 10°. The solution was allowed to warm to room temperature. The reaction was monitored by n.m.r. and was judged complete after 90 h. The solution was diluted with di-isopropyl ether (114 ml); washed with water, brine, and saturated aqueous sodium bicarbonate; dried over magnesium sulphate; and concentrated on a rotary evaporator at 80° to an oil, (99.0 g) which crystallized spontaneously. The crystalline mass was dissolved in isopropyl alcohol (140 ml) and a sample of the solution was diluted with DMSO-$d_6$ (2.5 volumes) for n.m.r. which showed that the *syn:anti* ratio was about 27:1. The rest of the solution was treated with pyridine (42 ml) and a solution of thiourea (38 g) in water (220 ml) at 18—25°, stirred for 5 h and allowed to stand overnight. The product was collected by filtration, washed with isopropyl alcohol/water (60 ml, 2:3), water (90 ml), and isopropyl alcohol (100 ml), and dried overnight *in vacuo* at 40° to give the title compound as the substantially pure *syn* isomer (88.2 g) as a solid; m.p. 191—194°; n.m.r. (DMSO-$d_6$) resembles that of Example 1.

**Claims**

1. A process for the preparation of a 4-halo-2-hydroxyiminoacetoacetic acid ester which comprises reacting a 4-halo-acetoacetic acid ester with an alkyl nitrite under acidic conditions.

2. A process according to claim 1 wherein the 4-halo-acetoacetic acid ester is a $C_{1-4}$ ester.

3. A process according to either of claims 1 and 2 wherein the halo substituent of the 4-halo-acetoacetic acid ester is a chlorine atom.

4. A process according to any one of the preceding claims wherein the alkyl nitrite is a $C_{1-6}$ alkyl nitrite.

5. A process according to claim 4 wherein the alkyl nitrite is isopropyl nitrite.

6. A process according to any one of the preceding claims wherein the 4-halo-2-hydroxyiminoacetoacetic acid ester obtained contains at least 75% of its *syn*-isomer.

7. A process according to any one of the preceding claims wherein the 4-halo-2-hydroxyiminoacetoacetic acid ester produced is reacted with thiourea to give a *syn* 2-(2-aminothiazol-4-yl)-2-hydroxyiminoacetic acid ester.

8. A process according to claim 7 wherein the 4-halo-2-hydroxyiminoacetoacetic acid ester is reacted without isolation with thiourea.

9. A process as claimed in claim 7 or 8 wherein the *syn* 2-(2-aminothiazol-4-yl)-2-hydroxyiminoacetic acid ester produced or a 2-(substituted oxyimino) derivative thereof is coupled with the amino group of a β-lactam nucleus to give a β-lactam antibiotic.

**Patentansprüche**

1. Verfahren zur Herstellung eines 4-Halogen-2-hydroxyiminoacetoessigsäureesters, der die Reaktion eines 4-Halogenacetoessigsäureesters mit einem Alkylnitrit unter sauren Bedingungen umfaßt.

2. Verfahren gemäß Anspruch 1, dadurch gekennzeichnet, daß der 4-Halogenacetoessigsäureester ein $C_{1-4}$-Ester ist.

3. Verfahren gemäß einem der Ansprüche 1 und 2, dadurch gekennzeichnet, daß der Halogensubstituent des 4-Halogenacetoessigsäureesters ein Chloratom ist.

4. Verfahren gemäß einem der vorhergehenden Ansprüche, dadurch gekennzeichnet, daß das Alkylnitrit ein $C_{1-6}$-Alkylnitrit ist.

5. Verfahren gemäß Anspruch 4, dadurch gekennzeichnet, daß das Alkylnitrit Isopropylnitrit ist.

6. Verfahren gemäß einem der vorhergehenden Ansprüche, dadurch gekennzeichnet, daß der

erhaltene 4-Halogen-2-hydroxyiminoacetoessigsäureester wenigstens 75% seines syn-Isomeren enthält.

7. Verfahren gemäß einem der vorhergehenden Ansprüche, dadurch gekennzeichnet, daß der erzeugte 4-Halogen-2-hydroxyiminoacetoessigsäureester mit Thioharnstoff umgesetzt wird, um einen syn-2-(2-Aminothiazol-4-yl)-2-hydroxyiminoessigsäureester zu ergeben.

8. Verfahren gemäß Anspruch 7, dadurch gekennzeichnet, daß der 4-Halogen-2-hydroxyiminoaceto-essigsäureester ohne Isolierung mit Thioharnstoff umgezetzt wird.

9. Verfahren gemäß Anspruch 7 oder 8, dadurch gekennzeichnet, daß der erzeugte syn-2-(2-Aminothiazol-4-yl)-2-hydroxyiminoessigsäureester oder ein 2-substituiertes Oxyiminoderivat davon mit der Aminogruppe eines β-Lactamrings gekuppelt wird, um ein β-Lactamantibiotikum zu ergeben.

**Revendications**

1. Procédé pour la préparation d'un ester d'acide 4-halogéno-2-hydroxyimino-acétoacétique, lequel comprend la réaction d'un ester d'acide 4-halogéno-acétoacétique avec un nitrite d'alkyle, dans des conditions acides.

2. Procédé selon la revendication 1, dans lequel l'ester d'acide 4-halogéno-acétoacétique est un ester en $C_{1-4}$.

3. Procédé selon l'une quelconque des revendications 1 et 2, dans lequel le substituant halogéno de l'ester d'acide 4-halogéno-acétoacétique est un atome de chlore.

4. Procédé selon l'une quelconque des revendications précédentes, dans lequel le nitrite d'alkyle est un nitrite d'alkyle en $C_{1-6}$.

5. Procédé selon la revendication 4, dans lequel le nitrite d'alkyle est le nitrite d'isopropyle.

6. Procédé selon l'une quelconque des revendications précédentes, dans lequel l'ester d'acide 4-halogéno-2-hydroxyimino-acétoacétique obtenu contient au moins 75% de son isomère *syn*.

7. Procédé selon l'une quelconque des revendications précédentes, dans lequel on fait réagir avec la thiourée l'ester d'acide 4-halogéno-2-hydroxyimino-acétoacétique produit, pour obtenir un ester d'acide 2-(2-aminothiazole-4-yl)-2-hydroxyiminoacétique *syn*.

8. Procédé selon la revendication 7, dans lequel on fait réagir avec la thiourée l'ester d'acide 4-halogéno-2-hydroxyimino-acétoacétique sans l'isoler.

9. Procédé selon la revendication 7 ou 8, dans lequel on couple l'ester d'acide 2-(2-aminothiazole-4-yl)-2-hydroxyiminoacétique *syn* produit, ou un dérivé 2-(oxyimino substitué) de celui-ci, avec le groupe amino d'un noyau β-lactame, pour obtenir un antibiotique de type β-lactame.